# EUROPÄISCHE PATENTSCHRIFT

(11) **EP 0 915 738 B1**
(45) Veröffentlichungstag und Bekanntmachung des Hinweises auf die Patenterteilung: **16.01.2002**
(21) Anmeldenummer: 97936669.7
(22) Anmeldetag: 29.07.1997
(51) Int. Cl.: B03C 1/00, B03C 1/30, A61M 5/31, A61K 49/00

(54) **VORRICHTUNG UND VERFAHREN ZUR ABTRENNUNG MAGNETISCHER MATERIALIEN AUS PHARMAZEUTISCHEN ZUBEREITUNGEN, DEREN AUSGANGS- ODER ZWISCHENPRODUKTEN SOWIE MIT HILFE DIESER VORRICHTUNG HERGESTELLTE MITTEL**
PROCESS AND DEVICE FOR SEPARATING MAGNETIC MATERIALS FROM PHARMACEUTICAL COMPOSITIONS, THEIR STARTING OR INTERMEDIATE PRODUCTS AND AGENTS PRODUCED BY MEANS OF THIS DEVICE
DISPOSITIF ET PROCEDE POUR LA SEPARATION DE MATERIAUX MAGNETIQUES CONTENUS DANS DES PREPARATIONS PHARMACEUTIQUES, LEURS PRODUITS INITIAUX OU INTERMEDIAIRES, AINSI QU'AGENTS OBTENUS A L'AIDE DE CE DISPOSITIF

(30) Priorität: 05.08.1996 DE 19632416
(43) Veröffentlichungstag der Anmeldung: 19.05.1999
(73) Patentinhaber: SCHERING AKTIENGESELLSCHAFT, 13353 Berlin (DE)
(72) Erfinder: WEITSCHIES, Werner, D-12105 Berlin (DE); RHEINLÄNDER, Thomas, D-12437 Berlin (DE); EBERT, Wolfgang, D-15831 Mahlow (DE); BETTER, Bernard, D-13404 Berlin (DE)
(86) Internationale Anmeldenummer: EP9704099
(87) Internationale Veröffentlichungsnummer: WO9805430

(56) Entgegenhaltungen:
- EP-A- 0 670 185
- WO-A-90/07380
- DE-A- 4 309 333
- US-A- 3 817 389
- PATENT ABSTRACTS OF JAPAN vol. 011, no. 185 (C-428), 13.Juni 1987 & JP 62 011519 A (AGENCY OF IND SCIENCE & TECHNOL;OTHERS: 01), 20.Januar 1987,
- PATENT ABSTRACTS OF JAPAN vol. 010, no. 112 (C-342), 25.April 1986 & JP 60 241914 A (HITACHI PLANT KENSETSU KK), 30.November 1985,

## Beschreibung

Die Erfindung betrifft den in den Patentansprüchen gekennzeichneten Gegenstand, das heißt ein Verfahren zur Herstellung von Kontrastmitteln für die Magnetresonanztomographie, sowie neue Kontrastmittel, die mit Hilfe dieses Verfahrens hergestellt werden.

In pharmazeutische Zubereitungen können durch Herstellungsgänge mit metallischen Werkzeugen oder in metallischen Behältern oder durch Spritzbestecke Fremdpartikel in Form metallischer partikulärer Verunreinigungen gelangen. Die Arzneibücher schreiben zum Schutz der Patienten deshalb für parenteral zu applizierende pharmazeutische Zubereitungen, hier insbesondere bei Infusionen, nach der Partikelgröße gewichtete Höchstgrenzen für die Anzahl von Fremdpartikeln vor. Häufig handelt es sich bei den Fremdpartikeln um ferro-, ferri-, superpara- oder paramagnetische Verbindungen.
Natürlich vorkommende ferromagnetische Verunreinigungen einer Ausgangssubstanz können nach einem Verfahren, das in der US-Patentschrift 4,119,700 beschrieben ist, abgetrennt werden. Hier werden die ferromagnetischen Verunreinigungen mit Hilfe eines Magnetfeldes abgetrennt. Aus den Offenlegungsschriften WO 90/07380 und WO 83/02405 sind Verfahren zur magnetischen Separation biologischer Materialien bekannt. Die Offenlegungsschrift WO 90/07380 beschreibt eine Vorrichtung, bei der ein Separationsraum von einem Permanentmagneten umgeben ist und der einen Zu- und Ablauf hat. Die europäische Patentanmeldung EP 670 185 beschreibt eine ähnliche Vorrichtung, mit deren Hilfe magnetisch markierte Zellen separiert werden.

Bei pharmazeutischen Zubereitungen wird bislang die Zahl der Fremdpartikel sofern irgend möglich durch Verfahren der Adsorptionsfiltration oder Membranfiltration erniedrigt. Insbesondere bei Verunreinigungen, die durch Manipulationen beim Anwender entstehen, wie z.B. Zuspritzung von Pharmaka in Infusionsbehältnisse, ist eine Reduktion der Anzahl der Fremdpartikel jedoch nur schwierig durchzuführen, da entsprechend kleinporige Membranfilter oft nur mit zusätzlichem mechanischem Druck betrieben werden können. Filtereinsätze in Infusionsbestecken weisen deshalb zumeist Porengrößen von einigen Mikrometern auf, die jedoch nur zu unbefriedigenden Rückhalteraten der Fremdpartikel führen. Bei partikulären pharmazeutischen Zubereitungen wie z.B. parenteralen Fettemulsionen oder Kristallsuspensionen als Depotarzneiformen ist eine Abtrennung von Fremdpartikeln durch Membran- oder Adsorptionsfiltration in der Regel überhaupt nicht möglich.

In der US-Patentschrift US 3,817,389 wird ein Filter beschrieben, der in ein Spritzbesteck integriert werden kann. Der Filter ist nicht magnetisch und enthält auch keine magnetischen Teilchen.

Es wurde ein Verfahren zur Herstellung von Kontrastmitteln für die Magnetresonanztomographie entwickelt, bei dem eine Partikelsuspension auf der Basis von para-, superpara-, ferro- oder ferrimagnetischen Partikeln.mit Hilfe eines magnetischen Filters filtriert wird.

Der magnetische Filter ermöglicht es, alle Verbindungen abzutrennen, die ferro-, ferri-, superpara- oder paramagnetisch sind.

Das zur Separation verwendete Gradientenfeld muß deutlich größer als der Gradient des Erdfeldes sein. Die Wahl des geeigneten Gradientenfeldes hängt von dem magnetischen Moment der abzutrennenden Substanz ab. Für die Separation paramagnetischer Verbindungen aus diamagnetischen pharmazeutischen Zubereitungen sind Hochgradientenfelder erforderlich.

Zur Abtrennung der unerwünschten magnetischen Verbindungen wird die jeweilige pharmazeutische Zubereitung oder deren Ausgangs- oder Zwischenprodukt durch den magnetischen Filter und somit durch ein magnetisches Gradientenfeld geleitet. Je höher der Gradient des magnetischen Gradientenfeldes ist, desto stärker ist die Kraft, die auf die para-, ferri-, superpara- oder ferromagnetischen Verunreinigungen wirkt. Arzneimittel und pharmazeutische Hilfsstoffe (wie zum Beispiel Wasser) sind in der Regel diamagnetisch und erfahren deshalb eine im Vergleich zu den para-, ferri-, superpara- oder ferromagnetischen Verunreinigungen sehr geringe Kraft, die sie im übrigen nicht in Richtung des Gradienten wandern läßt, sondern vielmehr abstößt. Für die Separation von magnetischen Verunreinigungen aus diamagnetischen Zubereitungen ist deshalb in der Regel bei der erfindungsgemäßen Separation im magnetischen Gradientenfeld im Gegensatz zur Filtration durch kleinporige Filter (z.B. 0,22 µm-Membranfilter) kein besonderer Druck auszuüben, es genügt in der Regel die Gravitationskraft beziehungsweise der hydrostatische Druck.

Die Abtrennung der unerwünschten magnetischen Partikel erfolgt bei dem erfindungsgemäßen magnetischen Filter mit Hilfe eines Durchflußverfahrens. Bei Durchflußverfahren muß, im Gegensatz zu statischen Verfahren, die Durchflußrate mit den magnetischen Momenten der zu separierenden ferro-, ferri- oder superparamagnetischen Substanzen und den angelegten Feldgradienten abgestimmt werden.

Die Ausführung des erfindungsgemäßen magnetischen Filters kann auf verschiedene Weise erfolgen. Das magnetische Gradientenfeld im Separationsraum kann beispielsweise durch einen Permanentmagneten oder einen Elektromagneten erzeugt werden, der außerhalb des Separationsraums angebracht ist. Zur Erhöhung des lokal wirksamen Gradienten des magnetischen Feldes kann es in diesem Fall von großem Nutzen sein, daß der Separationsraum aus paramagnetischem oder weichmagnetischem Material besteht und/oder paramagnetisches oder vorzugsweise weichmagnetisches Material enthält.

Es kann aber auch das magnetische Gradientenfeld im Separationsraum durch ein permanentmagnetisches Material erzeugt werden, welches den Separationsraum bildet oder sich im Separationsraum befindet.

Desweiteren kann das magnetische Gradientenfeld im Separationsraum durch einen stromdurchflossenen Leiter erzeugt werden, der sich entweder im Separationsraum befindet, oder den Separationsraum umgibt. In beiden vorgenannten Fällen kann es wiederum von großem Nutzen sein, daß der Separationsraum aus paramagnetischem oder weichmagnetischem Material besteht und/oder paramagnetisches oder vorzugsweise weichmagnetisches Material enthält.

Weichmagnetische Substanzen sind bevorzugt weichmagnetisches Eisen oder Stahl, insbesondere in Form von feinem Schrot (z.B. Kugeln mit wenigen Millimetern Durchmesser) oder Fritten oder in Form von Draht (wie z.B. Stahlwolle, Netze oder Siebe).

Die Wände des Separationsraumes sowie die weichmagnetischen oder paramagnetischen Materialien und die innerhalb des Separationsraumes sich befindenden stromduchflossenen Leiter können zum Schutz gegen unerwünschte chemische Reaktionen wie z.B. Korrosion zusätzlich mit geeigneten Schutzschichten versehen sein. Derartige Schutzschichten können die aus der Werkstoffkunde bekannten Materialien sein. Geeignet sind beispielsweise Verchromungen, Schutzschichten aus stabilen Oxiden (wie Aluminiumoxid), Kunststoffüberzüge (z.B.

PVC, Polystyrol, Polyethylen). Bei der Verwendung stromdurchflossener Leiter innerhalb der Separationskammer zur Erzeugung der magnetischen Gradientenfelder ist eine Isolierung mit den bekannten Isoliermaterialien (wie z.B. Kunststoffen in Form von Lacküberzügen) ohnehin erforderlich.

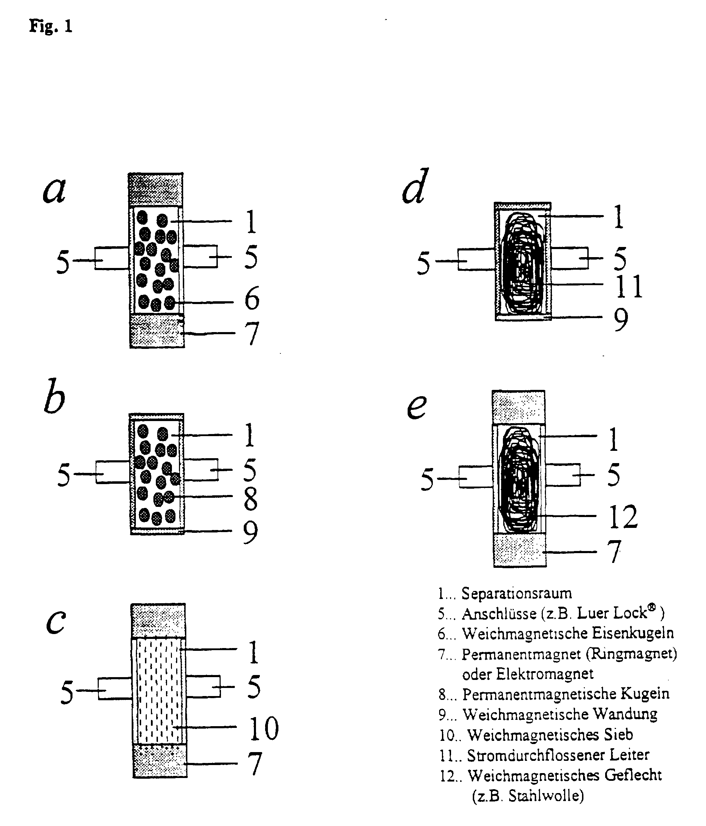

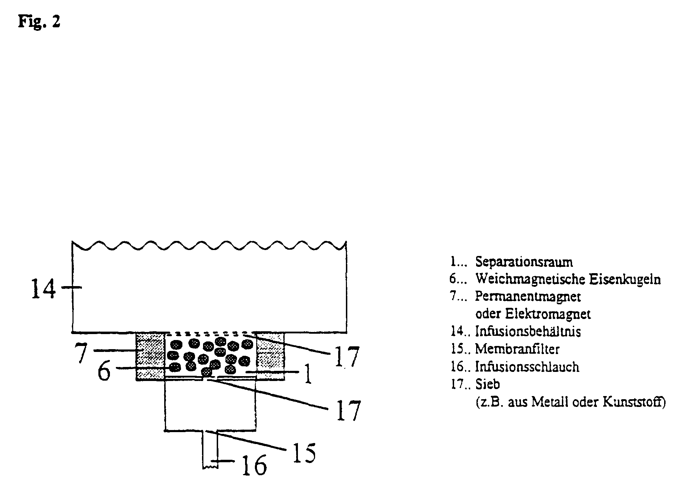

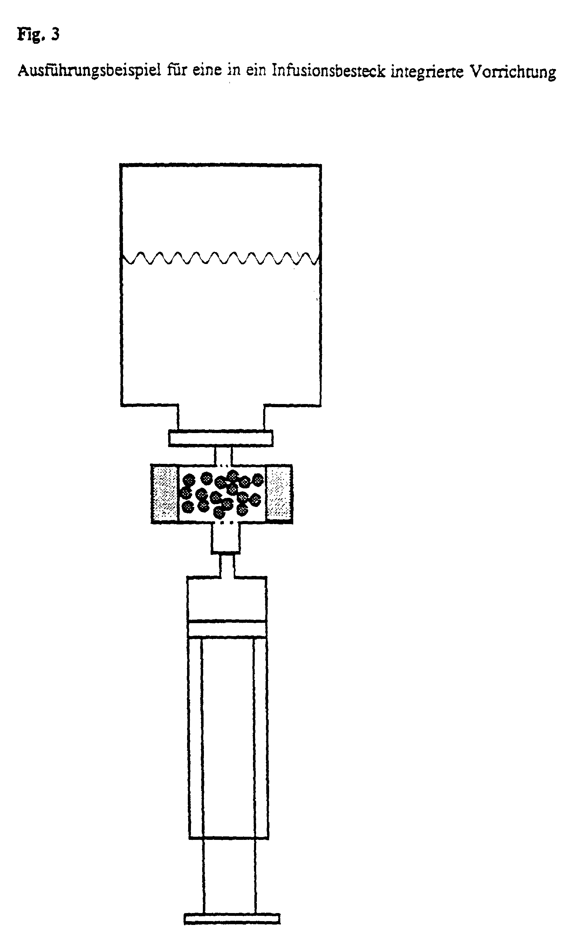

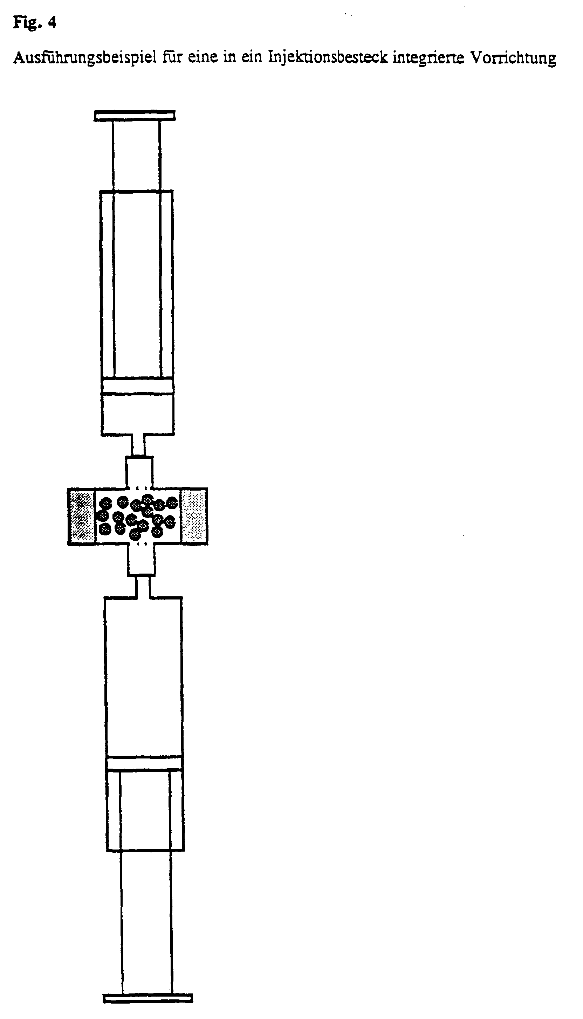

Beispiele für mögliche Ausführungsformen des erfindungsgemäßen magnetischen Filters als Vorsatzfilter sind in Figur 1 gezeigt. Der erfindungsgemäße magnetische Filter kann auch in Injektions- oder Infusionsbestecke integriert werden. Beispiele für in Infusionsbestecke integrierte Filter zur magnetischen Separation sind in Figur 2 und 3 dargestellt. Ein weiteres Ausführungsbeispiel für einen in ein Injektionsbesteck integrierten magnetischen Filter ist in Fig. 4 dargestellt. Die in Figur 1 skizzierten verschiedenen Ausführungsformen, wie zum Beispiel die Verwendung von permanentmagnetischen Kugeln oder stromdurchflossener Leiter, sind bei allen in Infusions- oder Injektionsbestecken integrierten erfindungsgemäßen Filtern zur magnetischen Separation anwendbar.

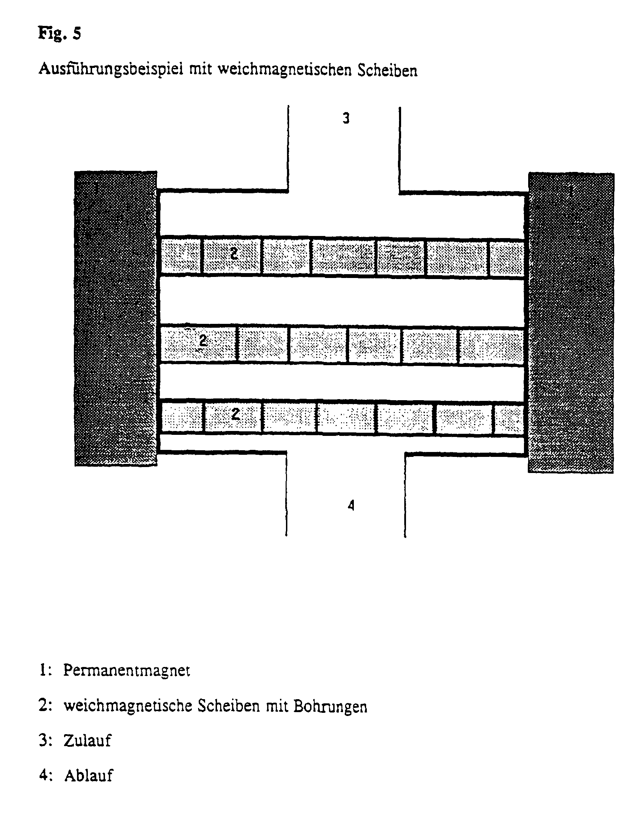

Eine weitere, spezielle Ausführungsform ist in Fig. 5 gezeigt. Hier enthält der Separationsraum eine weichmagnetische, während der Filtration zu magnetisierende Scheibe, die Bohrungen enthält, durch die die zu separierende Flüssigkeit bzw. die Suspension oder das Sol geleitet wird. Durch eine Vielzahl von Bohrungen mit kleinem Durchmesser kann eine sehr hohe magnetische Kontaktfläche für die zu separierende Flüssigkeit erreicht werden. Derartige Scheiben oder Zylinder werden bevorzugt aus Edelstahl gefertigt, wie er in pharmazeutischen Herstellprozessen verwendet wird. Sie können in Leitungssysteme integriert oder an solche adaptiert werden, was den besonderen Reinheitserfordernissen in einem pharmazeutischen Herstellungsprozeß vorteilhaft entspricht. Geeignete Edelstahlscheiben sind innerhalb großer pH-Bereiche korrosionsfest gegenüber aggressiven Arzneimittelbestandteilen. Desweiteren sind sie leicht zu reinigen, u.a. auch durch Anwendung der üblichen Hitzesterilisationsmethoden. Die Magnetisierung der Weichmagnetscheiben erfolgt durch einen Ringmagneten oder eine stromdurchflossene Spule, die sich außerhalb der Separationskammer befinden und damit nicht gereinigt werden müssen.
Durch den Durchmesser der Stahlscheiben, die Anzahl und Länge der Bohrungen, die der Höhe der Scheiben bzw. der Zylinder entspricht, kann die Fließgeschwindigkeit der zu separierenden Flüssigkeit und deren Verweildauer bzw. das Verhältnis der zu separierenden Flüssigkeit zur magnetisierten, benetzten Oberfläche in den Bohrungen so eingestellt werden, daß ein optimaler Separierungsgrad erfolgt. Desweiteren kann die Fließgeschwindigkeit in der Stahlscheibe bzw. im Zylinder reduziert werden, wenn zur Separierung mehrere Stahlscheiben oder Zylinder hintereinander verwendet werden und die Bohrungen der Scheiben oder Zylinder zueinander versetzt angeordnet sind.

Eine weitere spezielle Ausführungsform erhält man, wenn die Stahlscheiben im oberen Teil des Separationsraums nicht magnetisierbar sind bzw. aus nicht magnetisiertem Material bestehen und die Scheiben im unteren Teil des Separationsraums durch den äußeren Ringmagneten bzw. durch eine stromdurchflossene Spule magnetisiert werden. Dadurch wird erreicht, daß magnetische Partikel ausschließlich im unteren Teil des Separationsraums zurückgehalten werden.

Von besonderem Vorteil ist bei dem erfindungsgemäßen Filter zur magnetischen Separation pharmazeutischer Zubereitungen, daß dieser sich mit einfachen Mitteln wie zum Beispiel durch Hitzebehandlung, Autoklavieren mit gespanntem Wasserdampf und Begasung mit Ethylenoxid sterilisieren läßt. Desweiteren ist er viel stabiler als herkömmliche Membran- oder Porenfilter. Von besonderem Nutzen kann der erfindungsgemäße magnetische Filter auch als Vorfilter zur Reduktion der Partikelzahlen vor herkömmlichen Filtrationsverfahren wie zum Beispiel der Sterilfiltration sein.

Ein weiterer Aspekt der Erfindung betrifft die Herstellung von Kontrastmitteln, die mit Hilfe des erfindungsgemäßen magnetischen Filters erhältlich sind. Der erfindungsgemäße magnetische Filter eignet sich dazu, aus pharmazeutischen Zubereitungen auf der Basis von para-, superpara-, ferro- oder ferrimagnetischen Partikeln bestimmte Partikel zu selektieren.

Dieses kann über eine Variation der Feldstärke geschehen. So können aus einer pharmazeutischen Formulierung, die eine Mischung verschiedener magnetischer Teilchen enthält (wie z.B. eine Magnetit-haltige Suspension, wie sie in der Magnetresonanztomograpie Anwendung findet), die Teilchen abgetrennt werden, die ein besonders hohes magnetisches Moment aufweisen.

Magnetische Partikel enthaltende Mittel finden zum Beispiel Anwendung als Kontrastmittel in der Kernspintomographie. Dort werden u.a. Suspensionen auf der Basis von superparamagnetischen Magnetiten verwendet. Überraschenderweise ist es hier möglich, ein Partikelgemisch in Abhängigkeit unter anderem von der Gradientenfeldstärke nach seinen magnetischen Momenten aufzutrennen, d.h. hier kann das erfindungsgemäße Verfahren so gesteuert werden, daß nicht die vollständige Abscheidung der magnetischen Teilchen, sondern eine selektive Auftrennung erfolgt, wobei insbesondere die Teilchen mit hohen magnetischen Momenten zurückgehalten werden.

Die dabei erhaltenen Mittel weisen überraschenderweise erhebiich verbesserte Eigenschaften für ihren Anwendungszweck in der Diagnostik auf als die ursprünglichen Partikelsuspensionen. Damit werden Mittel für spezielle neue Anwendungsgebiete, wie z.B. für die Verwendung als Kontrastmittel in der Magnetresonanzangiograpnie oder Magnetresonanzlymphograhie, erhalten.

So kann mit Hilfe des erfindungsgemäßen magnetischen Filters Einfluß auf das Relaxationsverhalten der resultierenden Mittel und damit auf die Kontrastverstärkung in MRT-Verfahren Einfluß genommen werden. Für bestimmte medizinische Diagnosegeräte bzw. diagnostische Fragestellungen wird bevorzugt eine T₁-Relaxivitätsänderung, in anderen Fällen die T₂-Relaxivitätsänderung (oder eine geeignete Kombination der beiden) der Wasserstoffatome dem applizierten superparamagnetischen Partikeln benachbarten physiologischen Moleküle zur Erstellung einer Diagnose bzw. des diagnostischen Bildes herangezogen. Über die magnetische Separation kann auf diese Parameter - wie auch in den nachfolgenden Beispielen gezeigt - Einfluß genommen werden.

Mit Hilfe des magnetischen Filters ist somit die Herstellung eines Arzneimittels mit veränderten magnetischen Eigenschaften aus einem vorhandenen Arzneimittel möglich. Da die Aufnahme parenteral in den Menschen oder das Tier eingebrachter Partikel in das Retikuloendothelialen-System (RES) u.a. von deren Größe abhängt, erlaubt die magnetische Separation auch eine Einflußnahme auf die in vivo pharmakokinetischen Eigenschaften von pharmazeutischen Zubereitungen. Die bislang bekannten Methoden zur Steuerung der Größenverteilung sind unbefriedigend. Diese basieren auf aufwendigen, wenig steuerbaren Fällungsmethoden bei der Herstellung der Arzneimittelsubstanz oder auf Filtrationsprozessen. Letztere sind, wie bereits ausgeführt, mit immanenten Nachteilen behaftet.

Auch ein Abtrennen unerwünschter, vergleichsweise größerer superparamagnetischer Partikel aus kolloidalen Arzneimittelzubereitungen mit Zentrifugations- oder Sedimentationsprozessen ist verfahrenstechnisch äußerst aufwendig oder scheidet aus anderen Gründen, wie z.B. mangelnde Stabilität des Arzneimittels oder seiner Formulierung, aus.

Der erfindungsgemäße magnetische Filter wird weiterhin angewendet bei der Abtrennung von partikulären ferro- oder ferrimagnetischen Verunreinigungen aus paramagnetischen pharmazeutischen Zubereitungen, wie zum Beispiel Lösungen von Eisensalzen oder kolloidalem Eisendextran (z.B. *iron dextran injection,* USP XXV), die zur Therapie von Eisenmangelanämien eingesetzt werden.

Die nachfolgenden Beispiele dienen der näheren Erläuterung des Erfindungsgegenstandes, ohne ihn auf diese beschränken zu wollen.

### Beispiel 1

In 10 ml einer wäßrigen Lösung von 4,69 g Gadopentetsäure, Dimegluminsalz werden ca. 100 mg Eisenfeilspäne suspendiert. Ein magnetischer Filter, wie er in Figur le skizziert ist, wird aus einem Ringmagneten (RL 19, IBS Magnet Berlin, Außendurchmesser 19 mm, Innendurchmesser 6,5 mm, Höhe 10 mm) und einem im Innenvolumen des Ringmagneten angeordneten Separationsraum aufgebaut. Der Separationsraum besteht aus einer Wandung aus Kunststoff, und wird mit Stahlwolle gefüllt. Die Suspension wird im Durchfluß durch hydrostatischen Druck ohne zusätzliche Krafteinwirkung durch den magnetischen Filter gegeben. Nach der magnetischen Filtration kann durch mikroskopische Untersuchung gezeigt werden, daß die Eisenfeilspäne durch den Filter aus der Kontrastmittellösung abgetrennt werden.

### Beispiel 2

Ein magnetischer Filter, wie er in Abbildung la skizziert ist, wird aus einem Ringmagneten (NE 1556, IBS Magnet Berlin, Außendurchmesser 15 mm, Innendurchmesser 5 mm, Höhe 6 mm) und einem im Innenvolumen des Ringmagneten angeordneten Separationsraum aufgebaut. Der Separationsraum besteht aus einer Wandung aus Kunststoff, und wird mit Eisenschrotkugeln (Durchmesser ca. 0,3 mm) gefüllt. Durch den magnetischen Filter werden 0,8 ml einer superparamagnetischen kolloidalen Lösung von Eisenoxidnanopartikeln (hergestellt nach US 4,101,435; Beispiel 7 ) mit einem Eisengehalt von 500 mmol/1 und einer T₂-Relaxivity (r₂) von cirka 160 1 / (mmol s) mittels hydrostatischem Druck filtriert. Die T₂-Relaxivity (r₂) des Filtrats beträgt cirka 60 l / (mmol s).

Als Verhältnis für die Relaxivitäten r₂/r₁ wurde für die unbehandelte Lösung ein Wert von 7,4 bestimmt, wohingegen für das Filtrat ein Wert von 3,2 gemessen wurde.

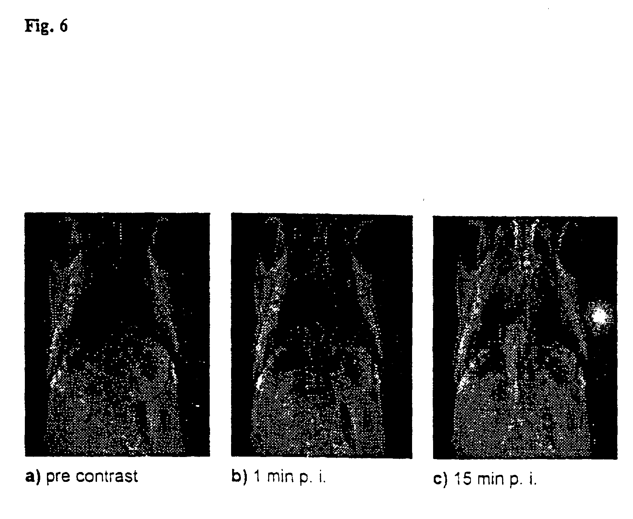

Alle MR-Angiogramme (Figur 6 - 8) wurden mittels A 3 D FLASH Technik (10/2,6/40°) auf einem experimental MRT (SISCO SIS 85, 2,0 Tesla) aufgenommen.

Als Versuchstiere wurden anesthesierte (Rompun®/Ketavet® Gemisch 1:1) Ratten (Han. Wistar;⁻ 200 g Körpergewicht) verwendet.

Sowohl mit der "unfiltrierten" Ausgangssubstanz als auch mit der erfindungsgemäßen "filtrierten" Zubereitung wurden zunächst jeweils eine Präkontrastaufnahme, sowie Aufnahmen 1, 15 bzw. 30 Minuten nach intravenöser Applikation des jeweiligen Kontrastmittels angefertigt. Dabei wurde stets eine Dosis von cirka 100 µmol Eisen / kg Körpergewicht verwendet.

Figur 6 zeigt die MR-Angiogramme der "unfiltrierten" Magnetit-Suspension. Der nach 1 Minute bzw. 15 Minuten erreichte Kontrasteffekt ist von geringem diagnostischen Aussagewert.

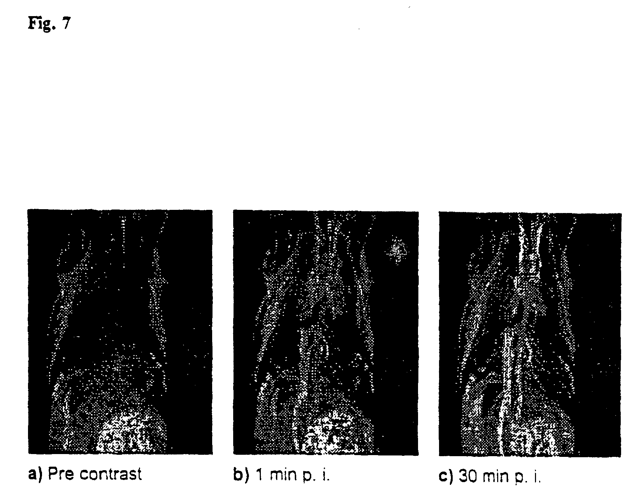

Figur 7 zeigt die MR-Angiogramme der "filtrierten" Magnetit Suspension [(a) Präkontrast, (b) 1 Minute p.i.), (c) 30 Minuten p.i.]. Hier ist bereits nach einer Minute eine große Anzahl von Gefäßen eindeutig detektierbar, der Effekt verstärkt sich noch dramatisch 30 Minuten nach Applikation des Kontrastmittels. Die erfindungsgemäß aufbereitete Kontrastmittlepräparation ist im Vergleich zur unbehandelten Substanz hervorragend für die Magnetresonanzangiographie geeignet.

### Beispiel 3

Ein magnetischer Filter, wie er in Abbildung la skizziert ist, wird aus einem Ringmagneten (NE 2016, IBS Magnet Berlin, Außendurchmesser 20 mm, Innendurchmesser 10 mm, Höhe 6 mm) und einem im Innenvolumen des Ringmagneten angeordneten Separationsraum aufgebaut. Der Separationsraum besteht aus einer Wandung aus Kunststoff, und wird mit Eisenschrotkugeln (Durchmesser ca. 0,3 mm) gefüllt. Durch den magnetischen Filter werden 0,8 ml einer superparamagnetischen kolloidalen Lösung von Eisenoxidnanopartikeln (hergestellt US 4,101,435; Beispiel 7) mit einem Eisengehalt von 500 mmol / l und einer T₂-Relaxivity (r₂) von cirka 160 l / (mmol s) mittels hydrostatischem Druck filtriert. Das Verhältnis der Relaxivitäten r₂ und r₁ beträgt beim Filtrat r₂/r₁ = 2,1.

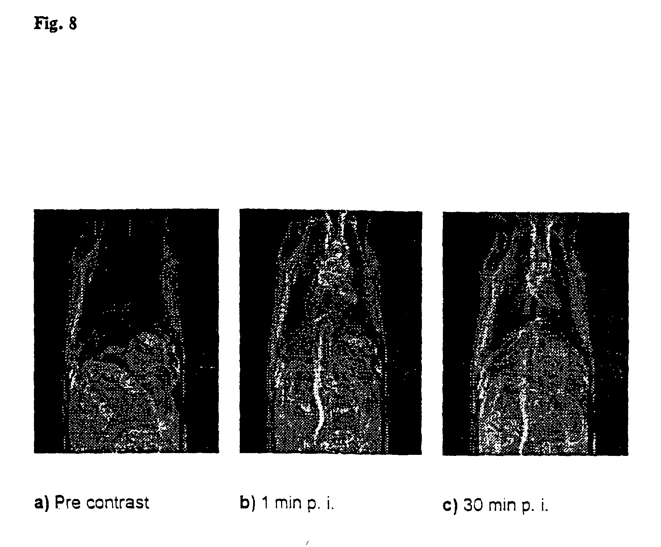

Die mit dieser Präparation erhaltenen Angiogramme sind in Figur 8 abgebildet, wobei bereits nach einer Minute eine Differenzierung der Gefäße erkennbar ist, wie sie bei der Präparation hergestellt nach Beispiel 2 erst zu einem deutlich späteren Zeitpunkt erreicht wird.

## Patentansprüche

1. Verfahren zur Herstellung von Kontrastmitteln für die Magnetresonanztomographie, **dadurch gekennzeichnet, daß** eine Partikelsuspension auf der Basis von para-, superpara-, ferro- oder ferrimagnetischen Partikeln mit Hilfe eines magnetischen Filters filtriert wird.

2. Verfahren gemäß Anspruch 1, **dadurch gekennzeichnet, daß** die Partikelsuspension eine Suspension auf der Basis von superparamagnetischen Magnetiten ist.

3. Verfahren gemäß Anspruch 1, **dadurch gekennzeichnet, daß** der magnetische Filter einen Separationsraum enthält, der einen Zu- und Ablauf hat, und in dem ein magnetisches Gradientenfeld herrscht.

4. Verfahren gemäß Anspruch 1 oder 3, **dadurch gekennzeichnet, daß** der magnetische Filter in Injektions- oder Infusionsbestecke integriert ist.

5. Verfahren gemäß Anspruch 3, **dadurch gekennzeichnet, daß** das magnetische Gradientenfeld im Separationsraum durch einen Permanentmagneten oder einen Elektromagneten erzeugt wird, der außerhalb des Separationsraumes angebracht ist.

6. Verfahren gemäß Anspruch 3 oder 5, **dadurch gekennzeichnet, daß** der Separationsraum aus paramagnetischem oder weichmagnetischem Material besteht und/oder paramagnetisches oder weichmagnetisches Material enthält.

7. Verfahren gemäß Anspruch 3, **dadurch gekennzeichnet, daß** das Gradientenfeld im Separationsraum durch permanentmagnetisches Material erzeugt wird, welches den Separationsraum bildet oder sich im Separationsraum befindet.

8. Verfahren gemäß Anspruch 3, **dadurch gekennzeichnet, daß** das magnetische Gradientenfeld im Separationsraum durch einen stromdurchflossenen Leiter erzeugt wird, der sich entweder im Separationsraum befindet oder den Separationsraum umgibt.

9. Verfahren gemäß Anspruch 7 oder 8, **dadurch gekennzeichnet, daß** der Separationsraum zusätzlich paramagnetisches oder weichmagnetisches Material enthält.

10. Verfahren gemäß Anspruch 1, **dadurch gekennzeichnet, daß** der magnetische Filter steril ist.

11. Verfahren gemäß Anspruch 3, **dadurch gekennzeichnet, daß** der Separationsraum eine oder mehrere weichmagnetische, während der Separation zu magnetisierende Scheiben enthält, welche mit Bohrungen versehen sind, durch die die zu separierende Flüssigkeit geleitet wird.

12. Verfahren gemäß Anspruch 11, **dadurch gekennzeichnet, daß** sich im Separationsraum mehrere Scheiben hintereinander befinden, deren Bohrungen zueinander versetzt angeordnet sind.

13. Verfahren gemäß Anspruch 11 oder 12, **dadurch gekennzeichnet, daß** die Scheiben im oberen Teil des Separationsraums nicht magnetisierbar sind bzw. aus nicht magnetisierbarem Material bestehen und die Scheiben im unteren Teil des Separationsraums durch einen äußeren Ringmagneten oder eine stromdurchflossene Spule magnetisiert werden.

14. Verfahren gemäß einem der Ansprüche 11 bis 13, **dadurch gekennzeichnet, daß** die weichmagnetischen Scheiben aus Edelstahl gefertigt sind.

15. Kontrastmittel für die Magnetresonanztomographie, **dadurch gekennzeichnet, daß** es nach dem Verfahren gemäß einem der Ansprüche 1 - 14 hergestellt wird.

16. Kontrastmittel für die Magnetresonanzangiographie, **dadurch gekennzeichnet, daß** es nach dem Verfahren gemäß einem der Ansprüche 1 - 14 hergestellt wird.

17. Kontrastmittel für die Magnetresonanzlymphographie, **dadurch gekennzeichnet, daß** es nach dem Verfahren gemäß einem der Ansprüche 1 - 14 hergestellt wird.

## Claims

1. Process for producing contrast agents for magnetic resonance tomography, **characterized in that** a particle suspension based on paramagnetic, superparamagnetic, ferromagnetic or ferrimagnetic particles is filtered with the aid of a magnetic filter.

2. Process according to Claim 1, **characterized in that** the particle suspension is a suspension based on superparamagnetic magnetites.

3. Process according to Claim 1, **characterized in that** the magnetic filter contains a separation chamber which has an admission and outlet and in which there is a magnetic gradient field.

4. Process according to Claim 1 or 3, **characterized in that** the magnetic filter is integrated in injection or infusion equipment.

5. Process according to Claim 3, **characterized in that** the magnetic gradient field in the separation chamber is generated by a permanent magnet or an electromagnet which is arranged outside the separation chamber.

6. Process according to Claim 3 or 5, **characterized in that** the separation chamber consists of paramagnetic or soft-magnetic material and/or contains paramagnetic or soft-magnetic material.

7. Process according to Claim 3, **characterized in that** the gradient field in the separation chamber is generated by permanent magnetic material which forms the separation chamber or is located in the separation chamber.

8. Process according to Claim 3, **characterized in that** the magnetic gradient field in the separation chamber is generated by a current-carrying conductor which either is located in the separation chamber or surrounds the separation chamber.

9. Process according to Claim 7 or 8, **characterized in that** the separation chamber additionally contains paramagnetic or soft-magnetic material.

10. Process according to Claim 1, **characterized in that** the magnetic filter is sterile.

11. Process according to Claim 3, **characterized in that** the separation chamber contains one or more soft-magnetic plates to be magnetized during the separation, which plates are provided with bores through which the fluid to be separated is conveyed.

12. Process according to Claim 11, **characterized in that** a plurality of plates are located one behind the other in the separation chamber and their bores are offset relative to one another.

13. Process according to Claim 11 or 12, **characterized in that** the plates in the upper part of the separation chamber are nonmagnetizable or are made of nonmagnetizable material, and the plates in the lower part of the separation chamber are magnetized by an outer ring magnet or a current-carrying coil.

14. Process according to one of Claims 11 to 13, **characterized in that** the soft-magnetic plates are made of special steel.

15. Contrast agent for magnetic resonance tomography, **characterized in that** it is produced using the process according to one of Claims 1 to 14.

16. Contrast agent for magnetic resonance angiography, **characterized in that** it is produced using the process according to one of Claims 1 to 14.

17. Contrast agent for magnetic resonance lymphography, **characterized in that** it is produced using the process according to one of Claims 1 to 14.

## Revendications

1. Procédé de fabrication d'agents de contraste pour tomographie par résonance magnétique, **caractérisé en ce qu'**une suspension particulaire à base de particules para-, superpara-, ferro- ou ferrimagnétiques est filtrée à l'aide d'un filtre magnétique.

2. Procédé selon la revendication 1, **caractérisé en ce que** la suspension particulaire est une suspension à base de magnétites superparamagnétiques.

3. Procédé selon la revendication 1, **caractérisé en ce que** le filtre magnétique contient un espace de séparation, qui a une amenée et une évacuation, et dans lequel règne un champ magnétique à gradient.

4. Procédé selon la revendication 1 ou 3, **caractérisé en ce que** le filtre magnétique est intégré aux trousses d'injection ou de perfusion.

5. Procédé selon la revendication 3, **caractérisé en ce que** le champ magnétique à gradient est produit dans l'espace de séparation par un aimant permanent ou un électroaimant qui est installé en dehors de l'espace de séparation.

6. Procédé selon la revendication 3 ou 5, **caractérisé en ce que** l'espace de séparation est constitué d'un matériau paramagnétique ou magnétique doux et/ou contient un matériau paramagnétique ou magnétique doux.

7. Procédé selon la revendication 3, **caractérisé en ce que** le champ à gradient dans l'espace de séparation est produit par un matériau magnétique permanent qui forme l'espace de séparation ou se trouve dans l'espace de séparation.

8. Procédé selon la revendication 3, **caractérisé en ce que** le champ magnétique à gradient dans l'espace de séparation est produit par un conducteur parcouru par un courant qui soit se trouve dans l'espace de séparation soit entoure l'espace de séparation.

9. Procédé selon la revendication 7 ou 8, **caractérisé en ce que** l'espace de séparation contient en outre un matériau paramagnétique ou magnétique doux.

10. Procédé selon la revendication 1, **caractérisé en ce que** le filtre magnétique est stérile.

11. Procédé selon la revendication 3, **caractérisé en ce que** l'espace de séparation contient un ou plusieurs disques magnétiques doux à magnétiser au cours de la séparation, qui sont pourvus d'orifices à travers lesquels le liquide à séparer est amené.

12. Procédé selon la revendication 11, **caractérisé en ce que** plusieurs disques, dont les orifices sont disposés de façon décalée l'un par rapport à l'autre, se trouvent dans l'espace de séparation l'un derrière l'autre.

13. Procédé selon la revendication 11 ou 12, **caractérisé en ce que** les disques de la partie supérieure de l'espace de séparation ne sont pas magnétisables ou selon le cas sont constitués d'un matériau non magnétisable et les disques de la partie inférieure de l'espace de séparation sont magnétisés par un aimant annulaire extérieur ou par une bobine parcourue par un courant.

14. Procédé selon l'une quelconque des revendications 11 à 13, **caractérisé en ce que** les disques magnétiques doux sont constitués d'acier allié.

15. Agent de contraste pour tomographie par résonance magnétique, **caractérisé en ce qu'**il est fabriqué par le procédé selon l'une quelconque des revendications 1-14.

16. Agent de contraste pour angiographie par résonance magnétique, **caractérisé en ce qu'**il est fabriqué par le procédé selon l'une quelconque des revendications 1-14.

17. Agent de contraste pour lymphographie par résonance magnétique, **caractérisé en ce qu'**il est fabriqué par le procédé selon l'une quelconque des revendications 1-14.
